# EUROPEAN PATENT APPLICATION

(11) **EP 1 535 998 A1**
(43) Date of publication of application: **01.06.2005**
(21) Application number: 03740519.8
(22) Date of filing: 15.07.2003
(51) Int. Cl.: C12N 9/16, C12N 15/55, C12N 15/82, C12Q 1/42

(54) **PLANT NUCLEOTIDE-SUGAR PYROPHOSPHATASE/PHOSPHODIESTERASE (NPPASE), METHOD OF OBTAINING SAME AND USE OF SAME IN THE PRODUCTION OF ASSAY DEVICES AND IN THE PRODUCTION OF TRANSGENIC PLANTS**

(30) Priority: 15.07.2002 ES 200201647; 20.11.2002 ES 200202673
(71) Applicant: UNIVERSIDAD PUBLICA DE NAVARRA, 31006 Pamplona (ES); Niigata University, Niigata City, Niigata Prefecture 950-2181 (JP); CONSEJO SUPERIOR DE INVESTIGACIONES CIENTIFICAS, 28006 Madrid (ES)
(72) Inventor: MUNOZ PEREZ, Francisco José, E-31006 Pamplona - Navarra (ES); RODRIGUEZ LOPEZ Milagros, E-31006 Pamplona - Navarra (ES); BAROJA FERNANDEZ, Miren Edurne, E-31006 Pamplona - Navarra (ES); POZUETA ROMERO, Francisco Javier, E-31006 Pamplona - Navarra (ES); MITSUI, Toshiaki, 8050 Ikarashi-2, Niigata 950-2181 (JP); NANJO, Yohei, Niigata, Niigata 950-2181 (JP)
(74) Representative: Elzaburu, Alberto de
(86) International application number: PCT/ES2003/000363
(87) International publication number: WO 2004/007706

(57) **Abstract**

NPPase is an enzyme that catalyses the hydrolysis of a wide range of small molecules with phosphodiester and phosphosulphate bonds, in particular ADPG (adenosine diphosphate glucose) and APS (adenosine 5'-phosphosulphate). The enzyme obtained from plant extracts is used in assay devices for determining levels of nucleoside diphosphate sugars, based either on the sugar-1-phosphate released, or on the nucleoside monophosphate, both of which are products formed by the reaction catalysed by NPPase, as well as the detection of sulphonucleotides such as 3'-phosphoadenosine 5'-phosphosulphate (PAPS) and APS. The amino acid sequence of the enzyme is also described, as well as the nucleotide sequence of a complete cDNA and another incomplete cDNA. Finally, it describes the production of transgenic plants that overexpress NPPase and that have a high content of sugars, low content of starch and cell-wall polysaccharides and high resistance to high concentrations of salts and high temperature.

## Description

### INDUSTRIAL FIELD TO WHICH THE INVENTION RELATES

The invention relates to the field of the production, purification and characterization of various isoforms of nucleotide pyrophosphatase/phosphodiesterase (NPPase) especially of rice and barley, and to the applications of this enzyme in the determination of levels of nucleotide sugars, and of sulphonucleotides, as well as in the production of transgenic plants in which there is overexpression of cDNA's of the genes that code for the said isoforms of NPPase, giving rise to plants with reduced content of starch and cell-wall polysaccharides and high resistance to salinity and temperature.

### STATE OF THE PRIOR ART

Starch is the principal storage form of carbohydrates in plants. It is accumulated in large quantities in organs such as seeds (wheat, barley, maize, pea, etc.) and tubers (potato and yam among others), and is a fundamental constituent of the human diet. On the other hand, starch is a polymer that is often used in the paper, cosmetic, pharmaceutical and food industries, and is also used as a basic component for the manufacture of biodegradable plastics and environment-friendly paints. Another polysaccharide, cellulose, is a fundamental component of the cell wall of plants, constituting the fundamental raw material in such important processes as papermaking. Consequently, investigation of the processes involved in the synthesis of these polymers of glucose is given priority in various areas of industrial production.

UDPglucose (UDPG) is the fundamental precursor of the biosynthesis of cellulose and of cell-wall polysaccharides. It is also the precursor molecule of processes connected with the glycosylation of proteins and lipids. On the other hand, ADPglucose (ADPG) is the universal precursor of the biosynthesis of starch in storage tissues of plants. Its concentration in the cell is determining for the quantity and quality of the starch produced by the plant. Considerations of the factors that govern the endogenous levels of ADPG and UDPG in the plant cell have basically revolved around their synthesizing enzymes, such as ADPG pyrophosphorylase, UDPG pyrophosphorylase and sucrose synthase (Preiss, (1988) "Biosynthesis of starch and its regulation". The Biochemistry of Plants. Vol. 14, Academic Press, New York, pp. 182-249; Pozueta-Romero, J., Perata, P., Akazawa, T. (1999) "Sucrose-starch conversion in heterotrophic tissues". Crit. Rev. Plant Sci. 18, 489-525). However, there has been little study of the mechanisms responsible for the degradation of these nucleotide sugars (Feingold, D.S., Avigad, G. (1980) "Sugar transformation in plants". The Biochemistry of Plants. Vol. 3, Stumpf, P.K. and Conn, E.E. eds. Academic Press, New York, pp. 101-170). There are indications that both bacteria and mammals possess enzymatic mechanisms capable of hydrolysing ADPG and UDPG (Melo, A., Glaser, L. (1966) "Nucleotide diphosphate hexose pyrophosphatases". Biochem. Biophys. Res. Commun. 22, 524-531; Bessman, M.J., Frick, D.N., O'Handley, S.F. (1996) "The MutT proteins or Nudix hydrolases, a family of versatile, widely distributed housecleaning enzymes". J. Biol. Chem. 271, 25059-25062; Rodriguez, P., Bass, S.T., Hansen, R.G. (1968) "A pyrophosphatase from mammalian tissues specific for derivates of ADP". Biochim. Biophys. Acta, 167, 199-201; Gasmi, L., Cartwright, J.L., McLennan, A.G. (1999) "Cloning, expression and characterization of YSA1H, a human adenosine 5'-diphosphosugar pyrophosphatase possessing a MutT motif". Biochem. J. 331-337; Moreno-Bruna, B., Baroja-Fernández, E., Muñoz, F.J., Bastarrica-Berasategui, A., Zandueta-Criado, A., Rodríguez-López, M., Akazawa, T., Pozueta-Romero, J. (2001) "Adenosine diphosphate sugar pyrophosphatase prevents glycogen biosynthesis in Escherichia coli". Proc. Natl. Acad. Sci. 98, 8128-8132). In plants, activity of this kind has barely been described in the literature (Salvucci, M.E., Crafts-Brandner, S.J. (1995) "Purification and properties of a unique nucleotide pyrophosphatase/phosphodiesterase I that accumulates in soybean leaves in response to fruit removal". Plant Physiol. 108, 1269-1276; Rodriguez-López, M., Baroja-Fernández, E., Zandueta-Criado, A., Pozueta-Romero, J. (2000) "Adenosine diphosphate glucose pyrophosphatase: a plastidial phosphodiesterase that prevents starch biosynthesis". Proc. Natl. Acad. Sci., 97, 8705-8710; Baroja-Fernández, E., Zandueta-Criado, A., Rodríguez-López, M., Akazawa, T., Pozueta-Romero, J. (2000) "Distinct isoforms of ADPglucose pyrophosphatase and ADPglucose pyrophosphorylase occur in the suspension-cultured cells of sycamore (*Acer pseudoplatanus* L.). FEBS Lett. 480, 277-282; Rodriguez-López, M., Baroja-Fernández, E., Zandueta-Criado, A. Moreno-Bruna, B. Muñoz, F.J., Akazawa, T., Pozueta-Romero, J. (2001) "Two isoforms of a nucleotide-sugar pyrophosphatase/phosphodiesterase from barley leaves (*Hordeum vulgare* L.) are distinct oligomers of HvGLP1, a germin-like protein". FEBS Lett. 490, 44-48).

In various industries, starch is a basic essential as a viscosity-increasing and gelling agent. The biosynthesis of starch in the plant cell starting from ADPG takes place in the subcellular compartment called the plastid. ADPG is both synthesized and utilized in this compartment, and therefore the levels of starch can be controlled by controlling the processes that regulate the ADPG levels. The various applications of starch produced in a plant are based on the balance of amylose and amylopectin, which determines the structure of the starch granule, as well as its viscosity in aqueous suspensions. The ratio of amylose to amylopectin depends on the concentration of ADPG in the plant cell. To date, no method is known for regulating the characteristics of the starch produced in a plant by controlling the degradation of ADPG, which the enzyme described in the present invention, can provide. In addition to acting as a storage substance for the plant, starch accumulates in the plant cell in circumstances where the plant is not subject to conditions of water stress. In conditions where the plant is subjected to high temperatures or high concentrations of salts in the environment, the plant stops accumulating starch, and produces large quantities of soluble sugars that accumulate in the vacuole (Keeling, P.L., Bacon, P.J., Holt, D.C. (1993) "Elevated temperature reduces starch deposition in wheat endosperm by reducing the activity of soluble starch synthase" Planta 191, 342-348; Geigenberger, P., Geiger, M., Stitt, M. (1998) "High-temperature perturbation of starch synthesis is attributable to inhibition of ADP-glucose pyrophosphorylase by decreased levels of glycerate-3-phosphate in growing potato tubers" Plant Physiol. 117, 1307-1316). In addition to these adaptive changes of carbohydrate metabolism to water stress, the plant undergoes changes in its sulphur metabolism, avoiding the accumulation of adenosine-5'-phosphate (PAP) arising from the transformation of adenosine 5'-phosphosulphate (APS) and 3'-phosphoadenosine 5'-phosphosulphate (PAPS) (Gil-Mascarell, R., López-Coronado, J.M., Bellés, J.M., Serrano, R., Rodriguez, P.L. (1999) "The Arabidopsis HAL2-like gene family includes a novel sodium-sensitive phosphatase" Plant J. 17, 373-383). On the basis of these observations, it is possible that enzymatic reactions responsible for the hydrolysis of ADPG, APS and PAPS are responsible for adaptive processes of the plant to the conditions of water stress.

Chromatographic and radiological techniques are powerful tools for determining levels of nucleotides such as sulphonucleotides (APS and PAPS among others; Yoshida, H., Fukui, S., Yamashina, I., Tanaka, T., Sakano, T., Usui, T., Shimotsuji, T., Yabuuchi, H.,

Owada, M., Kitagawa, T. (1982) "Elevation of nucleotide pyrophosphatase activity in skin fibroblasts from patients with Lowe's syndrome". Biochem. Biophys. Res. Commun. 107, 1144-1150) and nucleoside diphosphate sugars (such as the derivatives of glucose, ribose, mannose, galactose, glucuronic acid, fructose and galacturonic acid) in raw extracts of animal, plant or microbial origin. Although of very general use, they require a considerable investment in equipment and in the preparation of the test samples. Unfortunately, apart from a few exceptions (Puhakainen, E., Saarinen, A., Hänninen, O. (1977) "UDPglucuronic acid pyrophosphatase assay with the aid of alkaline phosphatase" Acta Chem. Scandinavica B31, 125-129) scant use is made of possible alternative methods that permit the detection and quantification of nucleotide sugars and sulphonucleotides in a simple and efficient manner. Analysis of the blood, muscle, kidney and liver levels of some of the aforementioned nucleotide sugars is important in clinical practice (Cortes, P., Dumler, F., Sastry, K.S., Verghese, C.P., Levin, N.W. (1982) "Effects of early diabetes on uridine diphosphosugar synthesis in the rat renal cortex". Kidney Int. 21, 676-682; Spiro, M.J. (1984) "Effect of diabetes on the sugar nucleotides in several tissues of the rat" Diabetologia 26, 70-75; Sochor, M., Kunjara, S., Baquer, N.Z., McLean, P. (1991) "Regulation of glucose metabolism in livers and kidneys of NOD mice". Diabetes 40, 1467-1471). For example, since UDPG is the precursor of glycogen in animals, analysis of the levels of this molecule can be important in the investigation and diagnosis of diseases associated with carbohydrate metabolism, such as various types of diabetes. On the other hand, determination of the levels of PAPS in the urine is essential for the diagnosis of serious illnesses such as Lowe's syndrome or antiphospholipid syndrome (Yoshida, H., Fukui, S., Yamashina, I., Tanaka, T., Sakano, T., Usui, T., Shimotsuji, T., Yabuuchi, H., Owada, M., Kitagawa, T. (1982) "Elevation of nucleotide pyrophosphatase activity in skin fibroblasts from patients with Lowe's syndrome". Biochem. Biophys. Res. Commun. 107, 1144-1150; Amigo, M.C., Garcia-Torres, T. (2000) "Morphology of vascular, renal, and heart lesions in the antiphospholipid syndrome: relationship to pathogenesis" Curr. Rheumatol. Rep. 2000, 2, 262-270). Obviously the possibility of analysing, simply and inexpensively, the levels of these substances in a sample represents an advantageous alternative to the chromatographic techniques.

The invention describes the purification and applications of an enzymatic product of plant origin that we designate NPPase, which catalyses the hydrolysis of small molecules with phosphodiester or phosphosulphate bonds and in particular ADPG and APS, as the preferred substrates. In the first priority (ES 200201647), with the experimental data available to them at that time, the inventors tentatively designated the enzymatic product isolated as NSPPase, but in the later, second priority (ES 200202673), with the data that had been accumulated, its designation was changed to NPPase as at present.

The plant enzyme of the invention has various isoforms in the plant tissues from which it can be obtained (Baroja-Fernández, E., Zandueta-Criado, A., Rodriguez-López, M., Akazawa, T., Pozueta-Romero, J. (2000) "Distinct isoforms of ADPglucose pyrophosphatase and ADPglucose pyrophosphorylase occur in the suspension-cultured cells of sycamore (*Acer pseudoplatanus* L.). FEBS Lett. 480, 277-282). The isoform that is simplest to extract is the one we call soluble, whereas other isoforms, which we can call particulates, are found firmly adhering to the starch granules.

In the present invention we succeeded in purifying and partially sequencing various soluble isoforms of NPPase of barley and rice with approximate sizes of 70 kDa. On the basis of these sequences, we were able to isolate the cDNA's that code for the NPPases. After comparing their sequences with those available in databases, it was observed that the NPPases of rice and barley share homology with PPD1, a nucleotide phosphatase/phosphodiesterase of *Lupinus luteus* which, in contrast to the NPPase of this invention, has as its best substrates the di- and tri-phosphate nucleosides but does not hydrolyse nucleotide sugars (Olczak, M., Olczak, T. (2002) "Diphosphonucleotide phosphatase/phosphodiesterase from yellow lupin (*Lupinus luteus* L.) belongs to a novel group of specific metallophosphatases". FEBS Lett. 519, 159-163). Moreover, it shares homology with other sequences that code for unknown or possible proteins of dicotyledonous plants such as Arabidopsis and chickpea. One object of the invention is, firstly, the production, characterization and sequencing of various soluble 70-kDa isoforms of NPPase in substantially pure form, starting from plant tissues of barley (*Hordeum vulgare*) and rice (*Oryza sativa*). Another object is the production of complete cDNA's that code for the NPPases and contrasting them with sequences available in databases. The design of constructions derived from the cDNA's of the soluble NPPases intended for the production of transgenic plants with high NPPase activity whose content and quality of the starch, as well as that of cell-wall polysaccharides, are modified relative to control plants, is detailed later. The said plants do not accumulate the marker of osmotic toxicity PAP, so that they are more resistant to high concentrations of salts than the control plants.

Another object of the invention is the method used for making devices or kits for determination of nucleoside diphosphate sugars and sulphonucleotides based on the use of the enzymatic product with NPPase activity.

### DETAILED DESCRIPTION OF THE INVENTION

The plant product with NPPase enzyme activity according to the invention can be obtained and purified starting from any plant tissue from any species, i.e. any monocotyledon or dicotyledon, for example barley (*Hordeum vulgare*), wheat (*Triticum aestivum*), rice (*Oryza sativa*), pepper (*Capsicum annuum*), tomato (*Lycopersicon sculentum*), potato (*Solanum tuberosum*), Arabidopsis (*Arabidopsis thaliana*), or sycamore (*Acer pseudoplatanus* L.), to cite just some of the numerous representative examples of different families and genera.

### Production and purification of a soluble isoform of NPPase:

The general method for obtaining and purifying the soluble plant NPPase described in the invention includes the following steps, in which slight variations are admissible that do not substantially alter the general scheme of the method of extraction and purification. NPPase is especially abundant in young leaves and is practically absent from storage tissues such as endosperms of seeds and tubers, accordingly it is recommended to use young leaves for the extraction of NPPase.
1. Homogenization of the plant tissue with an extraction buffer.
2. Filtration through four layers of Miracloth® (filter cloth for milk whey used in cheesemaking).
3. Ultracentrifugation of the filtered homogenate.
4. Precipitation of the proteins of the supernatant in ammonium sulphate.
5. Resuspension of the precipitate in buffer with pH of 4.2.
6. Heating for at least 15 minutes at a temperature between 60 and 65°C.
7. Centrifugation.
8. Concentration of the supernatant and purification of the protein by gel-filtration chromatography. The enzyme activity of NPPase is detected by detecting the production of G1P and AMP in samples incubated with substrates such as UDPG or ADPG.
9. Application of the NPPase to affinity column chromatography of the Concanavalin A type, which indicates that the NPPase is glycosylated.
10. Isoelectric focusing in a Multiphor II system, using PAGplates with pH range between 3.5 and 9.5 (Amersham/Pharmacia). The position of the NPPase can be determined easily in one of the following ways:
   a) Elution of the protein followed by detection of the production of G1P in the presence of ADPG or UDPG.
   b) Incubation of the gel in a solution with bis-paranitrophenylphosphate (bis-PNPP) and development in a basic solution as described by Nishimura and Beevers (Nishimura, M., Beevers, H. (1978) Plant Physiol. 62, 44-48).
11. Separation of the protein in denaturing gel by electrophoresis in a system of neutral or slightly acid buffers such as NuPAGE 4-12% Bis-Tris (Novex, San Diego, California). The position of the NPPase can be determined easily in one of the following ways:
   a) Elution of the protein followed by detection of the production of G1P in the presence of ADPG.
   b) Incubation of the gel in a solution with bis-PNPP and development in a basic solution.

### Identification of the product with NPPase enzyme activity

The enzyme product obtained by the methods described above, or other equivalent methods, is identified by means of the following functional standards:
- It is a pyrophosphatase/phosphodiesterase that catalyses the hydrolysis of ADPG in equimolar quantities of G1P and AMP (Rodríguez-López, M., Baroja-Fernández, E., Zandueta-Criado, A., Pozueta-Romero, J. (2000) "Adenosine diphosphate glucose pyrophosphatase: a plastidial phosphodiesterase that prevents starch biosynthesis". Proc. Natl. Acad. Sci., 97, 8705-8710)
- In addition to ADPG, it recognizes small molecules that possess phosphodiester and phosphosulphate bonds, such as UDPG, GDP-glucose, GDP-mannose, ADP-mannose, bis-PNPP, PAPS and APS and others of similar structure.
- It does not hydrolyse molecules with phosphomonoester bonds such as G1P, G6P, AMP, 3-phosphoglycerate, and other similar ones. Nor does it hydrolyse cyclic AMP or long-chain nucleic acids such as DNA or RNA, which are substrates of other phosphodiesterases described in the literature.
- In contrast to pyrophosphatases of ADP-sugars (EC 3.6.1.13, EC 3.6.1.21) described in bacteria and animals and in contrast to other phosphodiesterases (EC 3.1.4), its ionic requirements are reduced, therefore it can work in the absence of ions of magnesium, manganese, cobalt, and other divalent cations.
- In contrast to the pyrophosphatases of nucleoside diphosphate sugars of bacteria and animals, NPPase hydrolyses bis-PNPP.
- It is inhibited by phosphorylated molecules such as AMP, ADP, ATP, 3-phosphoglycerate, orthophosphate, inorganic pyrophosphate, and others with similar characteristics.
- It is strongly inhibited by molybdate and arsenate.
- It is resistant to ionic detergents such as SDS (sodium dodecylsulphate).
- It is resistant to the action of a wide range of proteases, such as Proteinase K and Pronase (Boehringer).
- Its activity is not affected by the action of typical inhibitors of phosphodiesterases such as β-mercaptoethanol, EDTA, reduced cysteine, ascorbate, and other reducing and chelating agents.
- It is sensitive to slightly basic pH and is very stable at pH between 4 and 7.5.

### Production of a complete cDNA that codes for the soluble NPPase

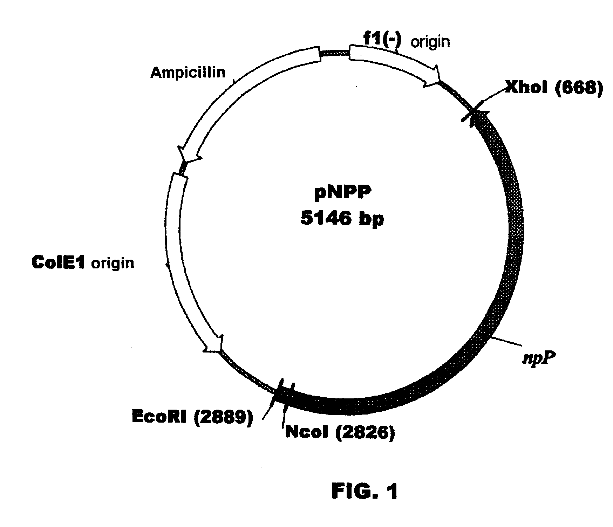

Some known internal amino acid sequences of the various NPPases purified were compared with others present in the databases. The result of this analysis made it possible to design two primers that were used for obtaining, by RT-PCR, a cDNA that codes for an NPPase of rice and of barley. The cDNA's obtained were cloned in the pGEM-T vector and were used as probes for searching for a complete cDNA in the cDNA library of young rice leaves. The complete cDNA obtained was introduced in the EcoRV restriction site of the plasmid pSK Bluescript (Stratagene) giving rise to the construction pNPP (Fig. 1) which was amplified in the host bacterium XL1 Blue. A strain of this bacterium was deposited on 15.10.02 in the Spanish Type Culture Collection, University of Valencia, Research Building, Campus of Burjasot, 46100 Burjasot, Valencia, with the number CECT 5739.

### Production of transgenic plants that overexpress the cDNA of soluble NPPase

pNPP was digested successively with the enzymes HindIII, T4 DNA polymerase and XbaI. The fragment released (which contains the cDNA of NPPase) was cloned in the plasmid pVT'BSP after being digested successively by the enzymes NcoI, T4 DNA polymerase and XbaI. In this way we obtain a plasmid designated p35SNPPNOS which has the constitutive promoter 35S, the cDNA of NPPase and the Nos terminator.

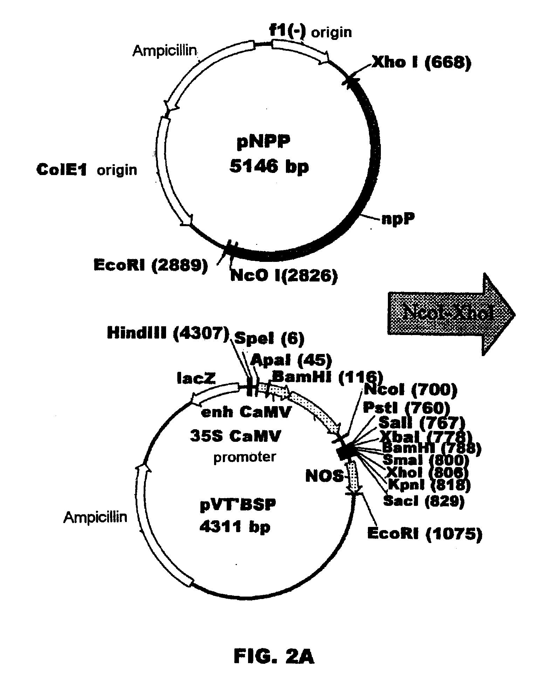

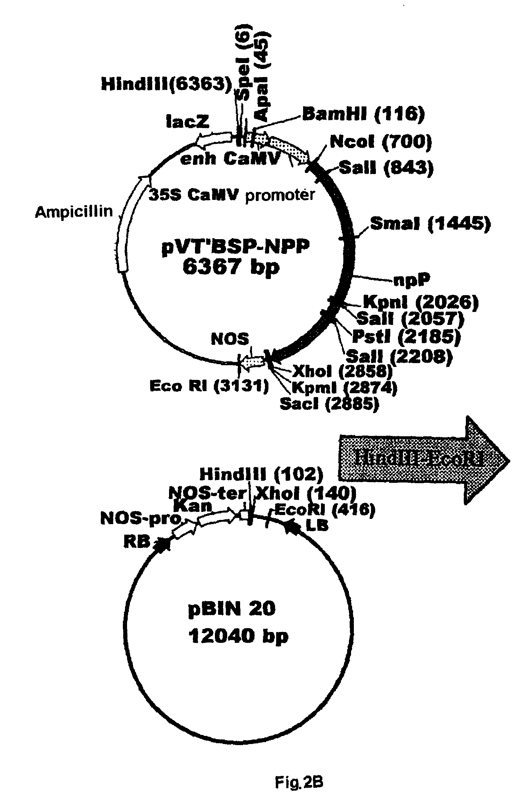

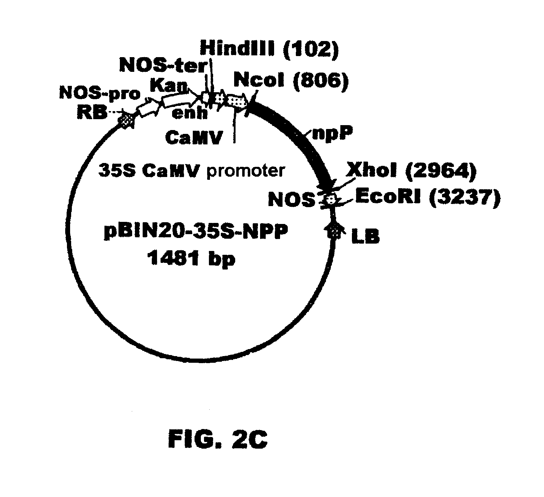

To be able to transfer this construction to the genome of the plants via *Agrobacterium tumefaciens*, it is necessary for it to be cloned previously in a binary plasmid. For this, p35SNPPNOS was digested successively with the enzymes HindIII, T4 DNA polymerase and XbaI and was cloned within the binary plasmid pBIN20 (Hennegan, K.P., Danna, K.J. "pBIN20: An improved binary vector for Agrobacterium-mediated transformation" Plant Mol. Biol. Rep. 16, 129-131) which had previously been digested successively with the enzymes HindIII, T4 DNA polymerase and XbaI. The plasmid thus obtained was designated with the name of pBIN20-35S-NPP (Fig. 2). After amplification in E. coli (XL1 Blue), pBIN20-35S-NPP was introduced into *Agrobacterium tumefaciens*, which was used for transforming species such as tomato, tobacco, potato etc. (Horsch, R.B., Fry, J.E., Hoffmann, N.L., Eichholtz, D., Rogers, S.G., Fraley, R.T. (1985) "A simple and general method for transferring genes into plants" Science 277, 1229-1231. The strain of *Agrobacterium tumefaciens* was deposited in the Spanish Type Culture Collection on 16.05.2003, located in the Research Building of the University of Valencia, Campus of Burjasot, Burjasot 46100 (Valencia, Spain) with the deposition number CECT 5799.

### Preparation of devices (assay kits) for determination of nucleoside diphosphate sugars and sulphonucleotides

The kits designed for the determination of nucleotides such as nucleoside diphosphate sugars and sulphonucleotides are based on the action of the product with NPPase activity on phosphodiester and phosphosulphate bonds of small molecules which, after being hydrolysed, give rise to other molecules that are easy to detect and quantify.

The two most suitable strategies for making these kits start from the hydrolysis of the nucleoside diphosphate sugar by the enzyme according to the present invention, i.e. NPPase, producing equimolar quantities of sugar-1-phosphate and of the corresponding nucleoside monophosphate. From here on, consideration can be given to determination of the quantity of nucleotide initially present in the sample based on determination of the quantity of sugar-1-phosphate and nucleoside monophosphate produced, as specified below:
- In the case when the sugar-1-phosphate is glucose-1-P (G1P), the said compound is submitted to the action of the enzyme phosphoglucomutase yielding glucose-6-phosphate, which in its turn can be made to undergo a coupling reaction with NAD⁺ by action of the enzyme glucose-6-phosphate dehydrogenase, obtaining 6-phosphogluconate and NADH, which is easily determined.
- In the case when the sugar-1-phosphate is not G1P, its determination and that of the nucleoside monophosphate take place by colorimetric determination of the orthophosphate (Pi) produced after the hydrolysis of these compounds with alkaline phosphatase.

Alternatively, as the coupling enzyme it is possible to use 5'-nucleotidase, which will hydrolyse the nucleoside monophosphate to equimolar quantities of the corresponding nucleoside and Pi. The Pi released in either of the two cases is easily quantifiable by known colorimetric methods.

The strategy for determination of levels of sulphonucleotides such as APS is based on the hydrolysis of these nucleotides and consequent production of equimolar quantities of sulphate, which can be determined by turbidimetry or by nephelometry (Sörbo, B. (1987) "Sulfate: turbidimetric and nephelometric methods" Methods Enzymol. 143, 3-6).

### Production of polyclonal antibodies specific to plant NPPase

Two milligrams of purified NPPase were separated in SDS-PAGE. After being eluted, it was mixed with complete Freund's adjuvant (at 50/50 ratio) and was then aliquoted in three equal fractions, each of which was injected into a rabbit at intervals of two weeks.

Approximately three months after the first injection, the blood serum of the rabbit was extracted, which contains the polyclonal antibodies specific to AGPPase.

### Identification of the product by Western blotting

Samples of proteins from wild plants and transgenic plants that overexpress the gene that codes for NPPase of rice were separated by SDS-PAGE. Then they were transferred to nitrocellulose membranes and the NPPase was detected using the specific anti-NPPase antibody according to the methodology described in the literature (Towbin, H., Staehelin, T., Gordon, J. (1979) "Electrophoretic transfer of proteins from polyacrylamide gels to nitrocellulose sheets: procedures and some applications" Proc. Natl. Acad. Sci. USA 76, 4350-4354.

### EXAMPLES OF APPLICATION OF THE INVENTION

Examples are presented below which describe in detail the method of production and purification of the soluble NPPase of young barley leaves. The same method, with slight changes appropriate to each case, can be applied to any other species. Other examples describe the use of NPPase for the production of assay kits for determination of nucleotide sugars and sulphonucleotides. Another example shows the production of a complete cDNA that codes for soluble NPPase.

### Example 1: Extraction and purification of the soluble NPPase obtained from barley leaves

All the steps were carried out at 4°C, unless indicated otherwise. The plant tissue (900 g) was homogenized with 2900 mL of extraction buffer (Mes 50 mM pH 6, EDTA 1 mM, DTT 2 mM) using a Waring blender. The homogenate was filtered through four layers of Miracloth, centrifuged at 100 000 g for 30 minutes and the supernatant was adjusted to 50% of ammonium sulphate.

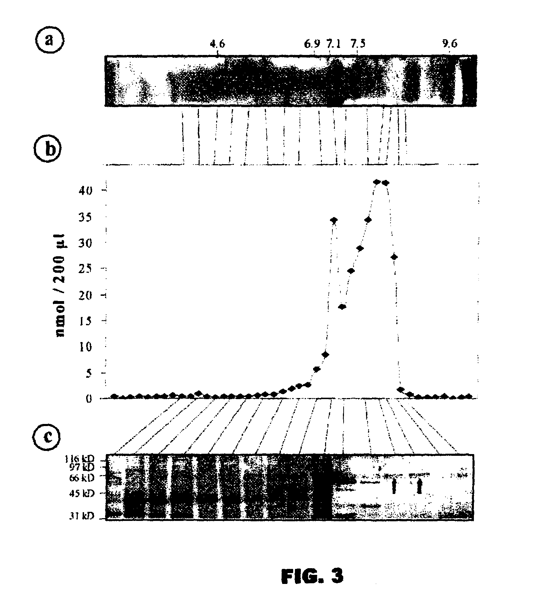

The precipitate obtained after 30 minutes of centrifugation at 30 000 g (20°C) was resuspended in 2900 mL of Mes 50 mM pH 4.2, then heated on a water bath at 62°C for 20 minutes, cooled in ice, and centrifuged at 30 000 g for 20 minutes. The proteins of the supernatant were precipitated using 50% ammonium sulphate, and resuspended in 5.7 mL of Mes 50 mM pH 6. Then the sample was submitted to gel filtration in a Superdex 200 column (Pharmacia LKB Biotechnology, Uppsala, Sweden) pre-equilibrated with Mes pH 6 and NaCl 150 mM. The fractions with NPPase activity were combined, concentrated, and submitted to isoelectric focusing in a native gel, permitting purification of the protein to homogeneity (Fig. 3).

### Example 2: Enzymatic tests

Unless stated otherwise, all the enzymatic reactions took place at 37°C. The determinations of NPPase activities were performed using spectrophotometric determination of G1P in two steps described by Sowokinos (1981) (Sowokinos, 1981, Plant Physiol. 68, 924-929). The reaction mixture contained Hepes 50 mM pH 7, the specified quantity of ADPG and the protein extract in a total volume of 50 microlitres. All the assays were performed relative to an ADPG blank. After 20 minutes of incubation, the reaction was stopped by boiling in a dry bath for 2 minutes. The mixture was centrifuged at 20 000 g for 5 minutes and the supernatant was recovered. In the second step, G1P was determined spectrophotometrically in 300 microlitres of mixture containing Hepes 50 mM pH 7, EDTA 1 mM, MgCl₂ 2 mM, KCl 15 mM, NAD⁺ 0.6 mM, one unit of phosphoglucomutase and one unit of glucose-6-phosphate dehydrogenase from *Leuconostoc mesenteroides*, and 30 microlitres of the supernatant resulting from step 1. After 20 minutes of incubation, the production of NADH was monitored at 340 nm using a Multiskan EX spectrophotometer (Labsystems). The amount of NADH produced by any protein extract in the absence of ADPG in step 1 was negligible.

The native molecular weight of the NPPase was determined by gel filtration by plotting the partition coefficient (Kav) against the logarithm of the molecular weight of the following protein standards: bovine thyroglobulin (670 kDa), bovine gamma-globulin (158 kDa), ovalbumin (45 kDa), myoglobin (17 kDa) and vitamin B-12 (1.3 kDa). The protein content was determined by Bradford's method using the reagent made by Bio-Rad and gamma-globulin as standard.

Table 1 presented below shows the purification of soluble NPPase starting from barley leaves. The unit (U) is defined as the quantity of enzyme that catalyses the production of 1 µmol of product per minute.

**Table 1**

| | Total volume (mL) | Total protein (mg) | Total activity (mU) | Specific activity (mU/mg protein) | Purification (factor) |
|---|---|---|---|---|---|
| Supernatant 100000 x g | 2900 | 24 286 | 1 195 000 | 50 | - |
| pH 4.2 / 62°C | 2900 | 778 | 1 067 000 | 1370 | 28 |
| Superdex 200 | 86 | 164 | 300 | 1820 | 37 |
| Isoelectric focusing | 14 | 0.6 | 21 870 | 36 455 | 740 |

### Example 3: Identification of the product with enzymatic activity obtained

The product with NPPase activity thus obtained complies with the following characteristics:
- It catalyses the hydrolysis of ADPG producing equimolar quantities of G1P and AMP.
- In addition to ADPG, the NPPase recognizes other small molecules that possess phosphodiester bonds, such as UDPG, GDP-glucose, bis-PNPP and others of similar structure. It also catalyses the hydrolysis of small molecules with phosphosulphate bonds, such as APS, releasing equimolar quantities of sulphate and AMP (Table 2 -Vmax in % in relation to ADPG- and Table 3).
- It does not hydrolyse molecules with phosphomonoester bonds such as G1P, G6P, AMP, 3-phosphoglycerate, and other similar molecules. Nor does it hydrolyse cyclic AMP or nucleic acids such as DNA and RNA, which are substrates of other phosphodiesterases described in the literature.
- Its ionic requirements are reduced, therefore the NPPase can work in the absence of ions of magnesium, manganese, cobalt, and other divalent cations, which are essential effectors for the functioning of other phosphodiesterases described in the literature.
- In contrast to the pyrophosphatases of nucleoside diphosphate sugars of bacteria and animals, NPPase hydrolyses bis-PNPP.
- It is inhibited by phosphorylated molecules such as AMP, ADP, ATP, 3-phosphoglycerate, orthophosphate, inorganic pyrophosphate, and others with similar characteristics.
- It is strongly inhibited by molybdate and arsenate.
- It is resistant to ionic detergents such as SDS (sodium dodecylsulphate).
- It is resistant to the action of a wide range of proteases, such as Proteinase K and Pronase (Boehringer).
- Its activity is not affected by the action of typical inhibitors of phosphodiesterases such as β-mercaptoethanol, EDTA, reduced cysteine, ascorbate, and other reducing and chelating agents.
- It is sensitive to slightly basic pH and is very stable at pH between 4 and 7.5. This characteristic is one that makes NPPase a completely different enzyme from the majority of the phosphodiesterases described in the literature, as the latter are stable and active at slightly basic pH values.
- It withstands a temperature of 65°C for 30 minutes, and can be characterized by the following data:

- Apparent molecular weight measured by gel filtration, around 70 kDa and 270 kDa, from which it is deduced that it has a monomeric form of 70 kDa and another homopolymeric form.
- K_{eq}' of the reaction 110.
- Increase in standard free energy (ΔG') of -2.9 kcal/mol.
- It is a glycoprotein, since it is retained by columns of concanavalin.
- Apparent molecular weight of the protein purified in denaturing gels, around 70 kDa.
- The amino acid sequences of barley NPPase obtained are:
   - N-terminal end: SEQ ID NO: 1
   - Internal sequences (obtained after partial hydrolysis of NPPase with trypsin): SEQ ID NO: 2, 3, 4, 5 and 6.
- The amino acid sequences of rice NPPase obtained are:
   - N-terminal end: SEQ ID NO: 7
   - Internal sequences (obtained after partial hydrolysis of NPPase with trypsin): SEQ ID NO: 8, 9, 10, 11, 12, 13, 14, 15, 16 and 17.

**Table 2:**

| Kinetic parameters and substrate specificity of NPPase from barley leaves | | |
|---|---|---|
| substrate | Km (mM) | Vmax |
| ADPG | 0.5 | 100 |
| ATP | 3.5 | 40 |
| ADP | 3.5 | 40 |
| APS | 0.5 | 160 |
| PAPS | - | - |
| PAP | n.q. | n.q. |
| ADPmannose | 0.4 | 30 |
| GDPmannose | 0.4 | 30 |
| CDPG | 2.8 | 114 |
| UDPG | 2.1 | 114 |
| ADPribose | 2.4 | 100 |
| NAD⁺ | 2.5 | 100 |
| NADP⁺ | n.q. | n.q. |
| bis-PNPP | 0.3 | 100 |
| PNPP | 0.5 | 100 |
| Hexose monophospates | n.q. | n.q. |
| Nucleotide monophosphates | n.q. | n.q. |

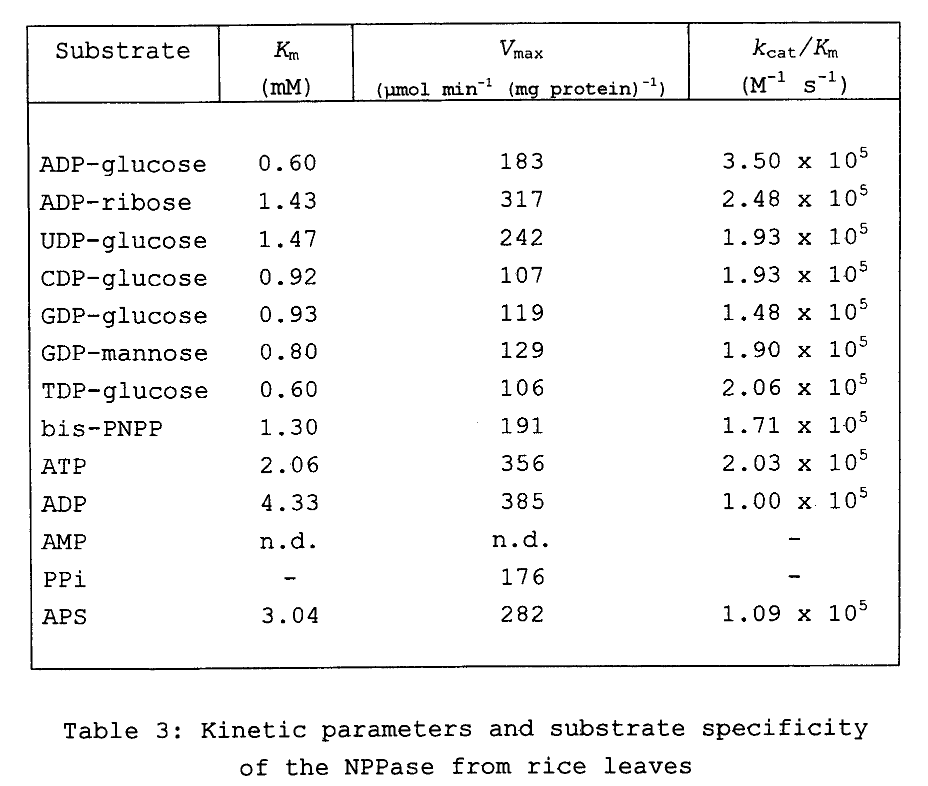

### Example 4: Obtaining a complete cDNA that codes for the soluble NPPase from rice and an incomplete cDNA that codes for the soluble NPPase of barley

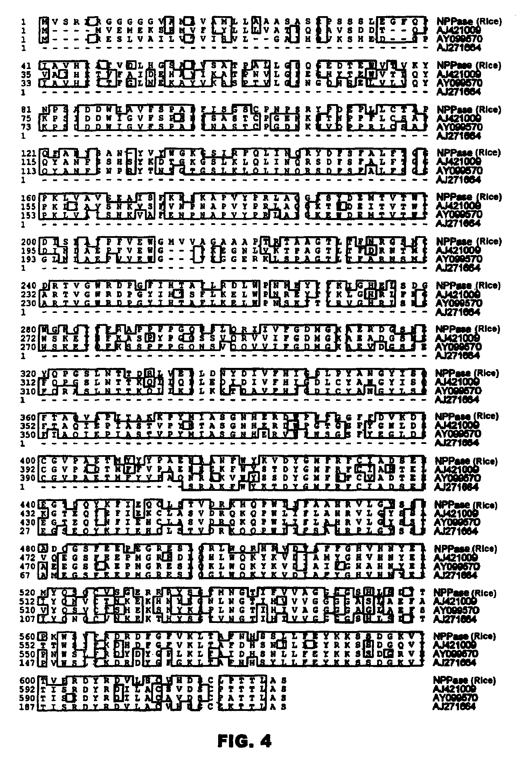

Knowledge of internal sequences of rice NPPase made it possible to design primers which, in the direction 5' - 3', are SEQ ID NO: 18 and 19. Using this primer, a cDNA was amplified by conventional methods of RT-PCR and was used as a probe for obtaining cDNA's from cDNA libraries from young leaves of rice and barley. As a result, a complete cDNA of rice NPPase was obtained and was inserted in a pSK Bluescript plasmid (Stratagene) which was amplified in the host bacterium XL1 Blue. The sequence of the complete cDNA of rice NPPase is SEQ ID NO: 20 and the amino acid deduced is SEQ ID NO: 21. An incomplete cDNA of barley NPPase was also obtained (SEQ ID NO: 22) and its deduced amino acid sequence is SEQ ID NO: 23. For this, the primer represented by SEQ ID NO: 24 was eliminated. After comparing with sequences available in databases, it was observed that the rice NPPase possesses 60% homology with PPD1 (access No. AJ421009), a nucleotide phosphatase/phosphodiesterase of *L. luteus* that hydrolyses nucleoside di- and triphosphates but is unable to hydrolyse nucleotide sugars (Olczak, M., Olczak, T. (2002) "Diphosphonucleotide phosphatase/phosphodiesterase from yellow lupin (*Lupinus luteus* L.) belongs to a novel group of specific metallophosphates". FEBS Lett. 519, 159-163). Moreover, as shown in Fig. 4, cDNA's were sequenced for dicotyledonous plants such as Arabidopsis (access No. AY099570) and chickpea (access No. AJ271664) which code for unknown or possible proteins that show high homology with the rice NPPase.

### Example 5: Products with NPPase activity from various plants

The NPPase enzyme exhibits a very wide distribution among plants, so that the enzymatic product with NPPase activity can be obtained from any plant, especially from tissues of young plants such as leaves and roots . For example, the following Table 4 shows the specific activities (mU / mg protein) obtained in various monocotyledons and dicotyledons.

**Table 4**

| | **Specific activity (mU / mg protein)** |
|---|---|
| | **(+ADPG)** |
| **Monocotyledons** | |
| Leaf of barley(*Hordeum vulgare*) | 113.7 ± 3.5 |
| Leaf of wheat (*Triticum aestivum*) | 22.4 ± 2.5 |

| **Dicotyledons** | |
|---|---|
| Leaf of *Arabidopsis thaliana* (Wt) | 5.2 ± 0.6 |
| Leaf of pepper (*Capsicum annuum*) | 5.0 ± 0.6 |
| Leaf of tomato *(Lycopersicon sculentum*) | 5.6 ± 0.5 |
| Cell culture of sycamore (*Acer pseudoplatanus*) | 16.5 ± 7.2 |

### Example 6: Preparation of enzyme kits for determination of glucose-nucleoside diphosphates

For the determination of glucose-nucleoside diphosphates such as ADPG, UDPG, CDP-glucose, GDP-glucose and TMP-glucose, a kit is prepared containing the following elements:
a. NPPase
b. NAD
c. Phosphoglucomutase (PGM)
d. G6P dehydrogenase (G6PDH)
e. Buffer

Determination of the quantity of glucose-nucleoside diphosphate present in the test sample is based on spectrophotometric determination of the NADH produced according to the following coupled reaction:

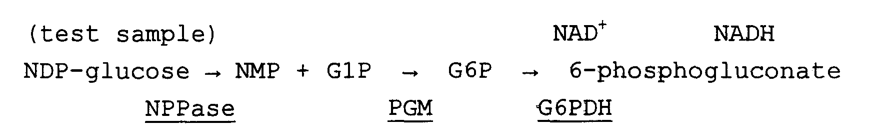

The quantity of NDP-glucose in a test sample could be determined by preparing a cocktail with the composition (for 1 ml):
- Test sample
- 1 U of NPPase
- 1 U of PGM
- 1 U of G6PDH
- 0.6 mM NAD
- Buffer Mes or Hepes 50 mM pH 7
- Water (make up to 1 ml)

Incubate at 37°C for 20 minutes and observe the change in absorbance of the sample at 340 nm. A cocktail not containing NPPase can be used as a negative control.

### Example 7: Preparation of enzyme kits for determination of nucleoside diphosphates of sugars other than glucose

Kits are prepared for determination of the following nucleoside diphosphate sugars:
- ribose-nucleoside diphosphates (ADP-ribose, GDP-ribose, UDP-ribose, CDP-ribose or TDP-ribose)
- mannose-nucleoside diphosphates (ADP-mannose, GDP-mannose, TDP-mannose, UDP-mannose or CDP-mannose)
- galactose-nucleoside diphosphates (ADP-galactose, GDP-galactose, UDP-galactose or CDP-galactose)
- glucuronic-nucleoside diphosphates (GDP-glucuronic, UDP-glucuronic, ADP-glucuronic, CDP-glucuronic or TDP-glucuronic)
- fructose-nucleoside diphosphates (GDP-fructose, ADP-fructose, CDP-fructose, UDP-fructose, TDP-fructose)
- galacturonic-nucleoside diphosphates (UDP-galacturonic, GDP-galacturonic, CDP-galacturonic, TDP-galacturonic or ADP-galacturonic)

The kit has the following elements:
a. NPPase
b. 5'-nucleotidase (or, alternatively, alkaline phosphatase)
c. buffer

Determination of the quantity of nucleoside diphosphate sugar present in the test sample is based on colorimetric determination of the orthophosphate released according to the following coupled enzymatic reaction:

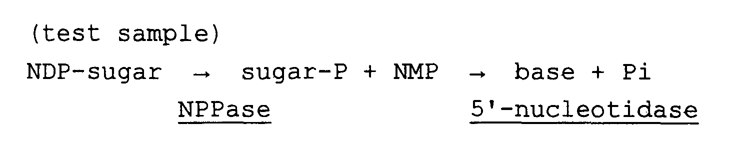

The Pi is determined according to any of the numerous colorimetric methods available in the bibliography and on the market.

The quantity of NDP-sugar in a test sample could be determined by preparing a cocktail (1 ml) consisting of:
- Test sample
- 1 U of NPPase
- 1 U of 5'-nucleotidase (or, alternatively, 1 U of alkaline phosphatase)
- Buffer Mes or Hepes 50 mM pH 7.5
- Water (make up to 1 ml)

Incubate at 37°C for 20 minutes and determine the production of Pi released according to conventional methods. A cocktail not containing NPPase can be used as negative control.

### Example 8: Preparation of enzyme kits for determination of PAPS and APS

The strategy for determination of levels of sulphonucleotides such as PAPS or APS is based on turbidimetric or nephelometric determination according to the following reaction:

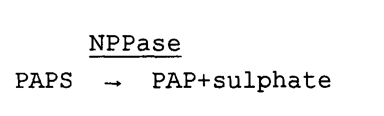

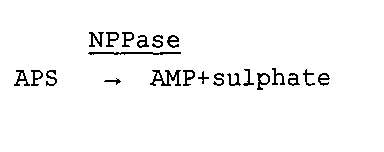

The quantity of sulphonucleotide in a test sample could be determined by preparing a cocktail (1 ml) consisting of:
- Test sample
- 1 U of NPPase
- Buffer Mes or Hepes 50 mM pH 7.0
- Water (make up to 1 ml)

Incubate at 37°C for 20 minutes and determine the production of sulphate released according to conventional methods. A cocktail not containing NPPase can be used as negative control.

### Example 9: Production of transgenic plants of tobacco, potato and tomato that overexpress NPPase

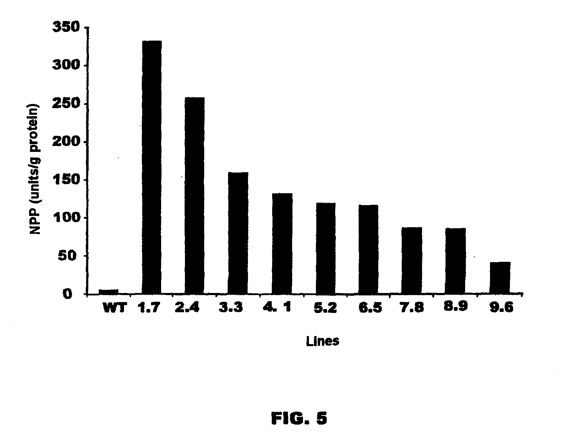

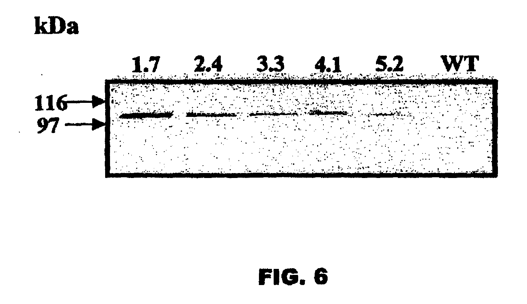

Using the strain of *Agrobacterium tumefaciens* CECT 5799, we obtained plants of tobacco (*Nicotiana tabacum*), potato (*Solanum tuberosum*) and tomato (*Lycopersicon sculentum*) with high NPPase activity in all the organs analysed (root, leaf, fruit and stalk) (Fig. 5). These plants accumulated large amounts of a protein that was recognized specifically by the polyclonal antibody obtained relative to rice NPPase (Fig. 6) and had the following characteristics:
1. Low content of starch and cell-wall carbohydrates (according to the measurement techniques based on commercial kits described in the literature (Frehner, M., Pozueta-Romero, J., Akazawa, T. (1990) "Enzyme sets of glycolysis, gluconeogenesis, and oxidative pentose phosphate pathway are not complete in nongreen highly purified amyloplasts of sycamore cell suspension cultures" Plant Physiol. 94, 538-544)).
2. High content of soluble sugars such as sucrose, glucose-6-phosphate, glucose and fructose.
3. Reduction of the levels of PAP accumulated in the tissues, imparting great resistance to high concentrations of sodium chloride in the growth substrate, relative to untransformed plants.
4. Resistance to high temperatures.

### DESCRIPTION OF THE DIAGRAMS:

Fig. 1: Schematic diagram of pNPP
Fig. 2 A-C: Production of pBIN20-35S-NPP
Fig. 3: Isolation of the 70-kDa NPPase by isoelectric focusing (IEF) in a negative gel:
   a) Corresponds to staining of the proteins after being separated by IEF, in relation to their isoelectric point (values shown at the top of the diagram). The cathode would be on the right and the anode on the left. A partially purified sample that contains 1 unit of AGPPase is applied to a plate of Ampholine PAG at a pH range 3.5-9.
   b) Corresponds to a profile of NPPase activity. The NPPase activity of each fraction eluted from the IEF gel is measured.
   c) Each fraction eluted from the IEF gel is submitted to SDS-PAGE and then staining with Coommassie-Blue. The fractions enriched with 70-kDa protein are indicated with an arrow and prove to be the most active enzymatically
Fig. 4: Comparison of amino acid sequences between the amino acid sequence deduced from the cDNA that codes for rice NPPase, and those deduced from the cDNA's that code for the PPD1 of Lupinus luteus (AJ421009) and unknown proteins of Arabidopsis (AY099570) and chickpea (AJ271664) .
Fig. 5: Hydrolytic activities of ADPglucose in wild-type (WT) and 9 transgenic lines of potato that overexpress rice NPP.
Fig. 6: Western blot of 5 transgenic lines of potato that overexpress rice NPP. 50 micrograms of protein were loaded in each lane and were submitted to SDS-PAGE. After transfer to nitrocellulose filters, the NPP was immunodecorated specifically after using the polyclonal antibody specific to NPPs obtained in the rabbit.

## Claims

1. A method of obtaining an enzyme product of plant origin with nucleotide sugar pyrophosphatase/phosphodiesterase activity (NPPase) in its soluble isoform, **characterized in that** the material of plant origin is submitted to extraction of the protein fraction by a buffer, filtration of the extract, followed by a method of purification by successive centrifugations and precipitations, with adjustments both of the pH and of the ionic strength of the medium, preferably including heating of the protein above 60°C and cooling in ice, and purification by gel filtration, isoelectric focusing, denaturing-gel electrophoresis, or other equivalent means of purification of proteins extracted from plant tissues.

2. The method as claimed in claim 1 comprising the following steps: (1) homogenization of the plant tissue with an extraction buffer, type Mes 50 mM pH 6, EDTA 1 mM, DTT 2 mM, (2) filtration, (3) ultracentrifugation at 100 000 g, (4) precipitation of the proteins of the supernatant in ammonium sulphate, (5) resuspension of the precipitate in buffer of pH 4.2, (6) heating for at least 15 minutes at a temperature between 60 and 65°C, followed by cooling in ice, (7) centrifugation at 30 000 g, (8) concentration of the protein of the supernatant by precipitation in ammonium sulphate and resuspension at pH 6, and (9) purification by gel-filtration chromatography, isoelectric focusing and denaturing-gel electrophoresis.

3. The enzyme product of plant origin obtainable by the method as claimed in claims 1 and 2, designated NPPase, **characterized in that** it is a phosphodiesterase that catalyses the hydrolysis of nucleotide sugars in equimolar conditions to sugar-phosphate and the corresponding nucleoside monophosphate, does not hydrolyse molecules with phosphomonoester bonds, is able to hydrolyse bis-PNPP, is inhibited by molybdate, arsenate and phosphorylated molecules, its activity is not affected by reducing and chelating agents that are inhibitors of phosphodiesterases, it is sensitive to slightly basic pH and is very stable at pH between 4 and 7.5, can be glycosylated, which makes it resistant to ionic detergents of the SDS type and to the action of proteases, and recognizes, in addition to nucleotide sugars, other small molecules that possess phosphodiester and phosphosulphate bonds.

4. The enzyme product as claimed in claim 3,
**characterized in that** it does not hydrolyse, among others, G1P, G6P, AMP, 3-phosphoglycerate, AMPc, nor nucleic acids.

5. The enzyme product as claimed in either one of the claims 3 and 4, **characterized in that** it does not require as effectors, among other divalent cations, magnesium, manganese or cobalt.

6. The enzyme product as claimed in any one of the claims 3 to 5, **characterized in that** it is inhibited by orthophosphate, inorganic pyrophosphate, and phosphate esters such as, among others, AMP, ADP, ATP, or 3-phosphoglycerate.

7. The enzyme product as claimed in any one of the claims 3 to 6, **characterized in that** its activity is not affected by, among others, β-mercaptoethanol, EDTA, reduced cysteine or ascorbate.

8. The enzyme product as claimed in any one of the claims 3 to 7, **characterized in that** it is resistant to, among others, Proteinase K or Pronase.

9. The enzyme product as claimed in any one of the claims 3 to 8, **characterized in that** it recognizes as substrates, among others, ADPG, UDPG, GDP-glucose, ADP-mannose, APS, PAPS or bis-PNPP, the preferred substrate being ADPG.

10. The enzyme product as claimed in any one of the claims 3 to 9, **characterized in that** it is resistant to a temperature of 65°C for 30 minutes, and **in that** it has an apparent molecular weight determined by gel filtration around 70 and 270 kDa for the monomeric and homopolymeric isoform respectively, as well as displaying a K_{eq}' of the reaction of 110, its ΔG' being -2.9 kcal/mol, and its Kₘ for ADPG being 0.5 mMolar.

11. The enzyme product as claimed in any one of the claims 3 to 10, **characterized in that** it was isolated from any plant species.

12. The enzyme product as claimed in any one of the claims 3 to 11, **characterized in that** its sequence contains at least one of the polypeptide fragments represented by SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8; SEQ ID NO: 9; SEQ ID NO: 10; SEQ ID NO: 11; SEQ ID NO: 12; SEQ ID NO: 13; SEQ ID NO: 14; SEQ ID NO: 15; SEQ ID NO: 16 and SEQ ID NO: 17.

13. The enzyme product as claimed in any one of the claims 3 to 12, **characterized in that** it is obtained from a cDNA that codes for a protein that contains at least one of the polypeptide sequences of claim 12.

14. Primers represented by SEQ ID NO: 18; SEQ ID NO: 19; SEQ ID NO: 24.

15. Use of primers represented by SEQ ID NO: 18; SEQ ID NO: 19; SEQ ID NO: 24 together with an mRNA from leaves of rice or barley for obtaining, by RT-PCR, a cDNA which, after being used as a probe on cDNA libraries of leaves of rice and barley, permits the isolation of cDNA's whose sequences are represented by SEQ ID NO: 20 and SEQ ID NO: 22, respectively.

16. cDNA represented by SEQ ID NO: 20 that codes for an enzyme product with NPPase activity.

17. Use of primers represented by SEQ ID NO: 18, SEQ ID NO: 19 or SEQ ID NO: 24 together with an mRNA from barley leaves for obtaining, by RT-PCR, a cDNA which, after being used as a probe on cDNA libraries of barley leaves, permits the isolation of cDNA whose sequence is represented by SEQ ID NO: 22.

18. cDNA represented by SEQ ID NO: 22 that codes for an enzyme product with NPPase activity.

19. Enzyme product, **characterized in that** it contains a sequence represented by SEQ ID NO: 21 with NPPase activity.

20. Enzyme product, **characterized in that** it contains a sequence represented by SEQ ID NO: 23 with NPPase activity.

21. Use of the enzyme product of claims 3 to 13 and 19 or 20 in the preparation of assay devices and/or compositions for application in the determination of nucleoside diphosphate sugars.

22. An assay device for the determination of nucleoside diphosphate sugars, **characterized in that** it includes the enzyme product of claims 3 to 13 and 19 or 20 in such a way that the determination is based on the sugar-1-phosphate released during the reaction catalysed by NPPase.

23. The assay device as claimed in claim 22, **characterized in that** the determination is based on the glucose-1-phosphate released, which is submitted to the enzyme phosphoglucomutase to produce glucose-6-phosphate, which in its turn is submitted to a coupled reaction with NAD⁺ and NADP⁺, by the action of the enzyme glucose-6-phosphate dehydrogenase, obtaining 6-phosphogluconate and NADH or NADPH, either of which can be determined by conventional spectrophotometric methods or methods of some other kind.

24. An assay device for the determination of nucleoside diphosphate sugars, **characterized in that** it includes the enzyme product of claims 3 to 13 and 19 or 20, in such a way that the determination is based on the nucleoside monophosphate produced during the reaction catalysed by NPPase.

25. The assay device as claimed in claim 24, **characterized in that** the determination is based on the nucleoside monophosphate, which is able to release orthophosphate, in addition to the corresponding base, by the action of an enzyme such as 5'-nucleotidase, the orthophosphate being easily determined by conventional methods, for example colorimetric methods.

26. The assay device as claimed in any of the claims 22 to 25, **characterized in that** the determination is based on the fact that the sugar-1-phosphate and the nucleoside-monophosphate are able to release orthophosphate by the action of an enzyme such as alkaline phosphatase or 5'-nucleotidase, the orthophosphate being easily determined by conventional methods, for example colorimetric methods.

27. Use of the enzyme product of claims 3 to 13 and 19 or 20 in the preparation of assay devices and/or compositions for application in the determination of the presence of 3'-phospho-adenosine 5'-phosphosulphate (PAPS) and adenosine 5'phosphosulphate (APS).

28. Use of the primers of claims 14 and the cDNA's of claims 16 or 18 in the production of transgenic plants that express or overexpress the cDNA that codes for NPPase.

29. Method of production of transgenic plants that express or overexpress the gene that codes for NPPase, **characterized in that** a transformation vector is used that contains a plasmid that includes the cDNA represented by SEQ ID NO: 19 of the gene of the said NPPase.

30. Method of production of transgenic plants that express or overexpress the gene that codes for NPPase, **characterized in that** it uses a transformation vector that contains a plasmid that includes the cDNA represented by SEQ ID NO: 22 of the gene of the said NPPase.

31. A method of production of transgenic plants as claimed in claim 29 or 30, **characterized in that** the transformation vector is *Agrobacterium tumefaciens* CECT 5799.

32. Transgenic plants obtainable by the method as claimed in claims 29 to 31, **characterized in that** they express or overexpress the enzyme product of claims 3 to 13 and 19 or 20 and have a reduced content of starch and/or of cell-wall polysaccharides and are resistant to high temperatures and to high salinity.

33. An assay device for the determination of sulphonucleotides, **characterized in that** it includes the enzyme product of claims 3 to 13 and 19 or 20 in such a way that the determination is based on the sulphate that is released.


SEQUENCE LISTING

<110> PUBLIC UNIVERSITY OF NAVARRA
<120> "Plant nucleotide sugar
5 pyrophosphatase/phosphodiesterase (NPPase), method of
production, use in the manufacture of testing devices
and in the production of transgenic plants"

<130>
<160>
<210> 1
<211> 16
<212> Peptide
<213> Hordeum vulgare cv. Scarlett
<220>
<223> N-terminal end of soluble NPPase
<400>
Ala Ala Val Arg Ala Ser Pro Asp Leu Leu Gly Ser Arg Gly Glu
                5                   10                  15
Asp
<210> 2
<211> 11
<212> Peptide
<213> Hordeum vulgare cv. Scarlett
<220>
<223> Tryptic sequence of soluble NPPase
<220> Variant
<222> 6
<223> / Nota = Lys
<220> Variant
<222> 9
<223> / Nota = Ile

<220> Variant
<222> 10
<223> / Nota = Lys

<400>
Ala Ser Tyr Pro Gly Gln Thr Ser Leu Gln Arg
                5                   10

<210> 3
<211> 11
<212> Peptide
<213> Hordeum vulgare cv. Scarlett
<220>
<223> Tryptic sequence of soluble NPPase
<220> Variant
<222> 9
<223> / Nota = Met

<400>
His Ala Pro Ala Asp Thr Val Thr Phe Gly Arg
                5                   10

<210> 4
<211> 5
<212> Peptide
<213> Hordeum vulgare cv. Scarlett
<220>
<223> Tryptic sequence of soluble NPPase

<400>
Ala Pro Pro Tyr Pro
                5

<210> 5
<211> 8
<212> Peptide
<213> Hordeum vulgare cv. Scarlett
<220>
<223> Tryptic sequence of soluble NPPase

<400>
Ala Trp Val Thr Val Glu Phe Lys
                5

<210> 6
<211> 8
<212> Peptide
<213> Hordeum vulgare cv. Scarlett
<220>
<223> Tryptic sequence of soluble NPPase
<220> Variant
<222> 1
<223> / Nota = Lys
<220> Variant
<222> 3
<223> / Nota = Ile
<220> Variant
<222> 6
<223> / Nota = Ile

<400>
Gln Ser Leu Glu Gly Leu Trp Arg
                5

<210> 7
<211> 15
<212> Peptide
<213> Oryza sativa
<220>
<223> N-terminal end of soluble NPPase

<400>
Gly Ser Ala Phe Val Ser Ala Thr Pro Ala Leu Leu Gly Asp Gln
                5                   10                  15
<210> 8
<211> 23
<212> Peptide
<213> Oryza sativa
<220>
<223> Tryptic sequence (MS/MS) of soluble NPPase

<400>
Phe Gln Leu Leu Asn Gln Arg Tyr Asp Phe Ser Phe Ala Leu Glu
                5                   10                  15
Thr Gly Gly Leu Glu Asn Pro Lys
                20

<210> 9
<211> 11
<212> Peptide
<213> Oryza sativa
<220>
<223> Tryptic sequence (MS/MS) of soluble NPPase

<400>
Leu Val Ala Val Ser Glu Ala Leu Ser Phe Lys
                5                   10

<210> 10
<211> 10
<212> Peptide
<213> Oryza sativa
<220>
<223> Tryptic sequence (Edman) of soluble NPPase

<400>
Leu Ala Gln Gly Lys Ser Tyr Asp Glu Met
                5                   10

<210> 11
<211> 10
<212> Peptide
<213> Oryza sativa
<220>
<223> Tryptic sequence (MS/MS) of soluble NPPase

<400>
Thr Ala Ala Gly Thr Leu Thr Phe Asn Arg
                5                   10

<210> 12
<211> 11
<212> Peptide
<213> Oryza sativa
<220>
<223> Tryptic sequence (MS/MS) of soluble NPPase

<400>
Asp Pro Gly Phe Leu His Thr Ala Phe Leu Arg
                    5                   10

<210> 13
<211> 12
<212> Peptide
<213> Oryza sativa
<220>
<223> Tryptic sequence (MS/MS) of soluble NPPase

<400>
Ala Pro Asp Phe Pro Gly Gln Asn Ser Leu Gln Arg
                    5                   10

<210> 14
<211> 9
<212> Peptide
<213> Oryza sativa
<220>
<223> Tryptic sequence (MS/MS) of soluble NPPase

<400>
Ile Ile Val Phe Gly Asp Met Gly Lys
                    5

<210> 15
<211> 12
<212> Peptide
<213> Oryza sativa
<220>
<223> Tryptic sequence (MS/MS) of soluble NPPase

<400>
Asp Trp Pro Asn Thr Gly Gly Phe Phe Asp Val Lys
                5                   10

<210> 16
<211> 11
<212> Peptide
<213> Oryza sativa
<220>
<223> Tryptic sequence (MS/MS) of soluble NPPase

<400>
Phe Ile Glu Gln Cys Leu Ser Thr Val Asp Arg
                5                   10

<210> 17
<211> 9
<212> Peptide
<213> Oryza sativa
<220>
<223> Tryptic sequence (MS/MS) of soluble NPPase

<400>
Val Tyr Asp Ser Phe Tyr Val Glu Arg
                5

<210> 18
<211> 18
<212> ADN
<213> Oryza sativa
<220>
<223> Primer of the 5' region of NPPase

<400>
ggcgttgctc ggcgacca

<210> 19
<211> 19
<212> ADN
<213> Oryza sativa
<220>
<223> Primer of the 3' region of NPPase

<400>
gaggcgagcg tggtgggga

<210> 20
<211> 2186
<212> ADN
<213> Oryza sativa
<220>
<223> complete cDNA of rice NPPase
<400>

ttt gaa tta gct agc tgt aga aag cta gcc 30
atg gtt agt agg aag aga gga gga gga gga 60
ggc gtg gca atg gcg gtg gcg atg ctg ctg 90
gcg gcg gcg agc gcg tcg cgg ccg tcg tcg 120
tcg ctg gaa ggg ttc cag ccg ctg tcg aag 150
atc gcc gtc cac aag gcc acc gtc gac ctc 180
cac ggc tcc gcg ttc gtc agc gcc acg ccg 210
gcg ttg ctc ggc gac cag gga gaa gac aca 240
gag tgg gtc acg gtg aaa tac ggc tgg gca 270
aac cct tcc gct gac gac tgg att gct gtc 300
ttc tct ccg gcc gat ttc atc tcg ggt tct 330
tgc cct aat cct tcc aga tac ccg gat gag 360
ccg ctg ctc tgc act gca cca ata aag tat 390
caa ttc gca aac tac tcg gcg aac tac gtg 420
tac tgg ggc aag ggc agc atc cgg ttc cag 450
ctc atc aac cag cgc tac gac ttc tcc ttc 480
gcc ctg ttc acc ggc ggc ctg gaa aac cct 510

aag ctg gtg gcg gtg tcg gag gcg ata tcg 540
ttc aag aac ccc aag gcg ccg gtg tac cct 570
cgg ctg gcg cag ggc aag tcg tac gac gag 600
atg acc gtc aca tgg acc agc ggc tac gac 630
atc agc gag gcg tac ccg ttc gtc gag tgg 660
ggc atg gtc gtc gcc ggc gcc gcc gct cca 690
acc cgc acc gcc gcc ggc acg ctc acc ttc 720
aac cgc ggc agc atg tgc ggt gac ccg gac 750
cgc act gtt ggg tgg aga gac ccc ggg ttc 780
atc cac aca gct ttc ctg aga gac ctg tgg 810
ccc aac aaa gag tac tac tac aag att ggg 840
cat gaa ctt tct gac gga tca att gtc tgg 870
ggc aag caa tac act ttc cgg gcg cca ccc 900
ttc cct ggc cag aac tcg ctg caa cgc atc 930
atc gtc ttc ggc gac atg ggc aag gcg gag 960
aga gac gga tca aac gag ttc gcc aac tac 990
cag cca gga tct ctg aac acg acg gac agg 1020
ctg gtc gag gat ctg gac aac tac gac att 1050
gtc ttc cac atc ggt gat ctt ccg tac gcc 1080
aat ggc tac atc tcc cag tgg gac cag ttc 1110
acc gcc cag gtc gcc ccc atc acc gcc aag 1140
aag ccc tac atg att gca agc ggt aac cat 1170
gag agg gac tgg ccc aac acc gga ggg ttc 1200
ttc gac gtc aag gac tcc ggc ggc gag tgc 1230
ggc gtt ccg gca gag acc atg tac tac tac 1260
ccg gcc gag aat cga gcc aac ttc tgg tac 1290
aag gtg gac tac ggg atg ttc cgg ttc tgc 1320
atc gcg gac tcg gag cac gac tgg agg gag 1350
ggt acc gac cag tac aag ttc atc gag cag 1380
tgc ctg tcg acg gtg gac cgg aag cac cag 1410
ccg tgg ctc atc ttc gcg gcg cac cgc gtg 1440
ctg ggc tac tcc tcc aac tgg tgg tac gcc 1470
gac cag ggc tcc ttc gag gag ccc gaa ggg 1500
agg gag agc ctg cag cgg ctg tgg cag cgc 1530
cac cgc gtc gac gtc gcc ttc ttc ggc cac 1560
gtc cac aac tac gag cgg acg tgc ccg atg 1590
tac cag agc cag tgc gtc tcc ggc gag agg 1620
cgc cgc tac tcc ggc acc atg aac ggc acc 1650
atc ttc gtc gtc gcc ggc ggc ggc ggg agc 1680
cac ctc tcg gac tac acc tcg gcg atc ccc 1710
aag tgg agc gtt ttc agg gac cgg gac ttc 1740
ggg ttc gtc aag ctc acc gcg ttc aac cac 1770
tcg tcg ctg ctg ttc gag tac aag aag agc 1800
agc gat ggg aag gtg tat gac tcc ttc acc 1830
gtg gag agg gat tac cgc gac gtg ctc agc 1860
tgc gtg cac gac agc tgc ctc ccc acc acg 1890
ctc gcc tcc tga tga atg aaa caa ggg aaa 1920
gga tca tta tta gga tgc atg agt tga tgc 1950

ctc atc gtc aaa atg ctg gca gct gag aaa 1980
gag tga tcg gtc ggt cga tcg agt tgg gtt 2010
tta ttt ttt ttc ttc ttc ttc aac cat ttc 2040
gat cag gtg tgg tag tgg tcg atc gct tgg 2070
ctc gat cgt gtt tct ctt cct cat gga tgg 2100
tga tgt tgt gca ata aaa ttg ctt agc tgc 2130
tcg ggc aca aat gtc taa aaa aaa aaa aaa 2160
aaa aaa aaa aaa aaa aaa aaa aaa aa 2186

<210> 21

<211> 623

<212> Peptide

<213> Oryza sativa

<220>

<223> Amino acid sequence of rice NPPase

<400>

Met Val Ser Arg Lys Arg Gly Gly Gly Gly Gly Val Ala Met Ala Val Ala Met Leu Leu
                5                   10                  15                  20
Ala Ala Ala Ser Ala Ser Arg Pro Ser Ser Ser Leu Glu Gly Phe Gln Pro Leu Ser Lys
                25                  30                  35                  40
Ile Ala Val His Lys Ala Thr Val Asp Leu His Gly Ser Ala Phe Val Ser Ala Thr Pro
                45                  50                  55                  60
Ala Leu Leu Gly Asp Gln Gly Glu Asp Thr Glu Trp Val Thr Val Lys Tyr Gly Trp Ala
                65                  70                  75                  80
Asn Pro Ser Ala Asp Asp Trp Ile Ala Val Phe Ser Pro Ala Asp Phe Ile Ser Gly Ser
                85                  90                  95                  100
Cys Pro Asn Pro Ser Arg Tyr Pro Asp Glu Pro Leu Leu Cys Thr Ala Pro Ile Lys Tyr
                105                 110                 115                 120
Gln Phe Ala Asn Tyr Ser Ala Asn Tyr Val Tyr Trp Gly Lys Gly Ser Ile Arg Phe Gln
                125                 130                 135                 140
Leu Ile Asn Gln Arg Tyr Asp Phe Ser Phe Ala Leu Phe Thr Gly Gly Leu Glu Asn Pro
                145                 150                 155                 160
Lys Leu Val Ala Val Ser Glu Ala Ile Ser Phe Lys Asn Pro Lys Ala Pro Val Tyr Pro
                165                 170                 175                 180
Arg Leu Ala Gln Gly Lys Ser Tyr Asp Glu Met Thr Val Thr Trp Thr Ser Gly Tyr Asp
                185                 190                 195                 200
Ile Ser Glu Ala Tyr Pro Phe Val Glu Trp Gly Met Val Val Ala Gly Ala Ala Ala Pro
                205                 210                 215                 220
Thr Arg Thr Ala Ala Gly Thr Leu Thr Phe Asn Arg Gly Ser Met Cys Gly Asp Pro Asp
                225                 230                 235                 240
Arg Thr Val Gly Trp Arg Asp Pro Gly Phe Ile His Thr Ala Phe Leu Arg Asp Leu Trp
                245                 250                 255                 260
Pro Asn Lys Glu Tyr Tyr Tyr Lys Ile Gly His Glu Leu Ser Asp Gly Ser Ile Val Trp
                265                 270                 275                 280
Gly Lys Gln Tyr Thr Phe Arg Ala Pro Pro Phe Pro Gly Gln Asn Ser Leu Gln Arg Ile
                285                 290                 295                 300
Ile Val Phe Gly Asp Met Gly Lys Ala Glu Arg Asp Gly Ser Asn Glu Phe Ala Asn Tyr
                305                 310                 315                 320
Gln Pro Gly Ser Leu Asn Thr Thr Asp Arg Leu Val Glu Asp Leu Asp Asn Tyr Asp Ile
                325                 330                 335                 340

Val Phe His Ile Gly Asp Leu Pro Tyr Ala Asn Gly Tyr Ile Ser Gln Trp Asp Gln Phe
                345                 350                 355                 360
Thr Ala Gln Val Ala Pro Ile Thr Ala Lys Lys Pro Tyr Met Ile Ala Ser Gly Asn His
                365                 370                 375                 380
Glu Arg Asp Trp Pro Asn Thr Gly Gly Phe Phe Asp Val Lys Asp Ser Gly Gly Glu Cys
                385                 390                 395                 400
Gly Val Pro Ala Glu Thr Met Tyr Tyr Tyr Pro Ala Glu Asn Arg Ala Asn Phe Trp Tyr
                405                 410                 415                 420
Lys Val Asp Tyr Gly Met Phe Arg Phe Cys Ile Ala Asp Ser Glu His Asp Trp Arg Glu
                425                 430                 435                 440
Gly Thr Asp Gln Tyr Lys Phe Ile Glu Gln Cys Leu Ser Thr Val Asp Arg Lys His Gln
                445                 450                 455                 460
Pro Trp Leu Ile Phe Ala Ala His Arg Val Leu Gly Tyr Ser Ser Asn Trp Trp Tyr Ala
                465                 470                 475                 480
Asp Gln Gly Ser Phe Glu Glu Pro Glu Gly Arg Glu Ser Leu Gln Arg Leu Trp Gln Arg
                485                 490                 495                 500
His Arg Val Asp Val Ala Phe Phe Gly His Val His Asn Tyr Glu Arg Thr Cys Pro Met
                505                 510                 515                 520
Tyr Gln Ser Gln Cys Val Ser Gly Glu Arg Arg Arg Tyr Ser Gly Thr Met Asn Gly Thr
                525                 530                 535                 540
Ile Phe Val Val Ala Gly Gly Gly Gly Ser His Leu Ser Asp Tyr Thr Ser Ala Ile Pro
                545                 550                 555                 560
Lys Trp Ser Val Phe Arg Asp Arg Asp Phe Gly Phe Val Lys Leu Thr Ala Phe Asn His
                565                 570                 575                 580
Ser Ser Leu Leu Phe Glu Tyr Lys Lys Ser Ser Asp Gly Lys Val Tyr Asp Ser Phe Thr
                585                 590                 595                 600
Val Glu Arg Asp Tyr Arg Asp Val Leu Ser Cys Val His Asp Ser Cys Leu Pro Thr Thr
                605                 610                 615                 620
Leu Ala Ser

<210> 22
<211> 1268
<212> ADN
<213> Hordeum vulgare cv. Scarlett
<220>
<223> incomplete cDNA of barley NPPase

<400>

ctc cga cgg aag cgt ggt gtg ggc caa gcc 30
cta cac ttt ccg ggc acc gcc aac ccc cgg 60
gca gaa ctc gct gca gcg tat cat cgt ctt 90
cgg tga cat ggg aaa ggc gga gag gga cgg 120
atc aaa cga gtt cgc caa cta cca gcc ggg 150
gtc gct caa cac gac gga cag gct gat tga 180
aga tct gga caa cta cga cat cgt ctt cca 210
cat cgg cga cat gcc cta cgc caa cgg gta 240
cct ctc cca gtg gga cca gtt cac cgc aca 270
ggt cgc ccc cat cag cgc caa gaa acc cta 300
cat ggt tgc aag cgg caa cca cga gag gga 330
ctg gcc caa cac cgg cgg gtt ctt cga cgt 360

caa gga ctc cgg cgg cga atg cgg cgt gcc 390
ggc cga gac cat gta cta cta ccc cgc cga 420
aaa cag ggc aaa ctt ctg gta caa ggt gga 450
cta cgg gat gtt ccg gtt ctg cgt ggg gga 480
ctc gga gca cga ctg gag gga ggg cac ccc 510
gca gta caa gtt cat cga gga gtg cct gtc 540
gac ggt gga ccg gaa gca cca gcc gtg gct 570
cat ctt cac ggc gca ccg ggt gct ggg cta 600
ctc ctc caa ctc gtg gta cgc cga cca ggg 630
ctc ctt cga gga gcc cga ggg acg gga gag 660
cct gca gaa gct gtg gca gcg cta ccg cgt 690
cga cat cgc ctc ctt cgg cca cgt cca caa 720
cta cga gcg cac atg ccc gct cta cca gag 750
cca gtg cgt caa cgc cga caa gac cca cta 780
ctc ggg cac cat gaa cgg cac cat ctt cgt 810
cgt cgc cgg cgg ggg cgg cag cca cct gtc 840
gtc cta cac cac cgc cat ccc caa gtg gag 870
cat att cag gga cca tga cta cgg gtt cac 900
caa gct cac cgc att caa cca ctc ctc gct 930
tct ctt cga gta cat gaa gag cag cga cgg 960
caa ggt cta cga ctc ctt cac cat cca cag 990
gga tta ccg cga cgt gct cag ctg cgt gca 1020
cga cag ctg ctt ccc cac cac gct cgc tag 1050
cta gct cat atc gtc cgg ccg tca tgt caa 1080
tgt aat gga ggg tca tcc atc caa taa aat 1110
tgt ggg cat gtg ttg agt aat aaa att ggt 1140
cag ctg cac aat tta tat gtg cta gta aaa 1170
aga tca tgc aag agg tgg gtg tat gct cgt 1200
tat ata tgc ttt gta act cct tca tgt cat 1230
att att atg ggt taa taa aaa cat cct tta 1260
tta aaa aa 1268

<210> 23
<211> 350
<212> Peptide
<213> Hordeum vulgare cv. Scarlett
<220>
<223> Amino acid sequence deduced from the cDNA of barley NPPase

<400>

Ser Asp Gly Ser Val Val Trp Ala Lys Pro Tyr Thr Phe Arg Ala Pro Pro Thr Pro Gly
                5                   10                  15                  20
Gln Asn Ser Leu Gln Arg Ile Ile Val Phe Gly Asp Met Gly Lys Ala Glu Arg Asp Gly
                25                  30                  35                  40
Ser Asn Glu Phe Ala Asn Tyr Gln Pro Gly Ser Leu Asn Thr Thr Asp Arg Leu Ile Glu
                45                  50                  55                  60
Asp Leu Asp Asn Tyr Asp Ile Val Phe His Ile Gly Asp Met Pro Tyr Ala Asn Gly Tyr
                65                  70                  75                  80
Leu Ser Gln Trp Asp Gln Phe Thr Ala Gln Val Ala Pro Ile Ser Ala Lys Lys Pro Tyr
                85                  90                  95                  100
Met Val Ala Ser Gly Asn His Glu Arg Asp Trp Pro Asn Thr Gly Gly Phe Phe Asp Val
                105                 110                 115                 120
Lys Asp Ser Gly Gly Glu Cys Gly Val Pro Ala Glu Thr Met Tyr Tyr Tyr Pro Ala Glu
                125                 130                 135                 140
Asn Arg Ala Asn Phe Trp Tyr Lys Val Asp Tyr Gly Met Phe Arg Phe Cys Val Gly Asp
                145                 150                 155                 160
Ser Glu His Asp Trp Arg Glu Gly Thr Pro Gln Tyr Lys Phe Ile Glu Glu Cys Leu Ser
                165                 170                 175                 180
Thr Val Asp Arg Lys His Gln Pro Trp Leu Ile Phe Thr Ala His Arg Val Leu Gly Tyr
                185                 190                 195                 200
Ser Ser Asn Ser Trp Tyr Ala Asp Gln Gly Ser Phe Glu Glu Pro Glu Gly Arg Glu Ser
                205                 210                 215                 220
Leu Gln Lys Leu Trp Gln Arg Tyr Arg Val Asp Ile Ala Ser Phe Gly His Val His Asn
                225                 230                 235                 240
Tyr Glu Arg Thr Cys Pro Leu Tyr Gln Ser Gln Cys Val Asn Ala Asp Lys Thr His Tyr
                245                 250                 255                 260
Ser Gly Thr Met Asn Gly Thr Ile Phe Val Val Ala Gly Gly Gly Gly Ser His Leu Ser
                265                 270                 275                 280
Ser Tyr Thr Thr Ala Ile Pro Lys Trp Ser Ile Phe Arg Asp His Asp Tyr Gly Phe Thr
                285                 290                 295                 300
Lys Leu Thr Ala Phe Asn His Ser Ser Leu Leu Phe Glu Tyr Met Lys Ser Ser Asp Gly
                305                 310                 315                 320
Lys Val Tyr Asp Ser Phe Thr Ile His Arg Asp Tyr Arg Asp Val Leu Ser Cys Val His
                325                 330                 335                 340
Asp Ser Cys Phe Pro Thr Thr Leu Ala Ser
                345                 350

<210> 24

<211> 39

<212> ADN

<213> Hordeum vulgare cv. Scarlett

<220>

<223> Primer of the 5' region of NPPase                    soluble

<400>

gcagcagtac gagcatcacc agatctacta gcatcacga        39